(19) Europäisches Patentamt
European Patent Office
Office européen des brevets

(11) Veröffentlichungsnummer: **0 007 402**
**B1**

(12) **EUROPÄISCHE PATENTSCHRIFT**

(45) Veröffentlichungstag der Patentschrift: **12.05.82**

(51) Int. Cl.³: **C 07 D 339/06**, A 01 N 43/28

(21) Anmeldenummer: **79101771.8**

(22) Anmeldetag: **05.06.79**

(54) **2-Oxo- und 2-Thioxo-1,3-dithiol-Verbindungen, ihre Herstellung und Verwendung und sie enthaltende, den Pflanzenmetabolismus regulierende Wirkstoffe.**

(30) Priorität: **07.06.78 CH 6224/78**

(43) Veröffentlichungstag der Anmeldung: **06.02.80 Patentblatt 80/3**

(45) Bekanntmachung des Hinweises auf die Patenterteilung: **12.05.82 Patentblatt 82/19**

(84) Benannte Vertragsstaaten: **CH DE FR GB IT**

(56) Entgegenhaltungen:
**DE-B-1 643 495**
**FR-A-1 510 016**
**US-A-3 187 009**

(73) Patentinhaber: **CIBA-GEIGY AG, Patentabteilung Postfach, CH-4002 Basel (CH)**

(72) Erfinder: **Farooq, Saleem, Dr., Im Schaiengarten 2, CH-4107 Ettingen (CH)**

(74) Vertreter: **Zumstein sen., Fritz, Dr. et al, Bräuhausstrasse 4, D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 007 402 B1

# 2-Oxo- und 2-Thioxo-1,3-dithiol-Verbindungen, ihre Herstellung und Verwendung und sie enthaltende, den Pflanzenmetabolismus regulierende Wirkung

Die vorliegende Erfindung betrifft 2-Oxo- und 2-Thioxo-dithiol-Verbindungen mit den Pflanzenmetabolismus regulierender Wirkung, Verfahren zu ihrer Herstellung, sie als Wirkstoffe enthaltende Mittel sowie deren Verwendung zur Regulierung des Pflanzenmetabolismus, insbesondere zur selektiven Förderung der Ausbildung von Trenngeweben an Früchten und Blättern von Pflanzen zur Erleichterung der Ernte.

Die neuen 2-Oxo-1,3-dithiol- und 2-Thioxo-1,3-dithiol-Verbindungen entsprechen der Formel I

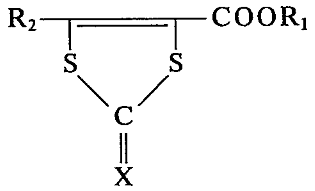

(I)

worin

$R_1$ $C_1-C_8$-Alkyl, gegebenenfalls ein- oder mehrfach durch Sauerstoff unterbrochen oder substituiert durch Halogen, Phenyl oder Phenoxy, wobei der Phenylkern durch Halogen, Cyano, Nitro, Trifluormethyl, $C_1-C_4$-Alkyl, $C_1-C_4$-Alkoxy oder Phenoxy substituiert sein kann;
$C_3-C_8$ Alkenyl, gegebenenfalls substituiert durch Halogen;
$C_3-C_8$ Alkinyl;
$C_3-C_6$ Cycloalkyl;
Phenyl, gegebenenfalls substituiert durch Halogen, Cyano, Nitro, Trifluormethyl, $C_1-C_4$-Alkyl, $C_1-C_4$-Alkoxy oder Phenoxy, $C_1-C_4$-Alkoxycarbonyl, Acetyloxy oder Dioxy-$C_1-C_2$-alkylen;

$R_2$ Wasserstoff, Methyl; $C_1-C_8$-Alkoxycarbonyl oder Phenyl, gegebenenfalls substituiert durch Halogen, Cyano, Nitro, Trifluormethyl, $C_1-C_4$-Alkyl, $C_1-C_4$-Alkoxy oder Phenoxy;

X Sauerstoff oder Schwefel bedeuten, mit der Maßgabe, daß wenn $R_1$ Methyl ist, $R_2$ nicht Wasserstoff oder Methoxycarbonyl sein kann.

Strukturähnliche Verbindungen sind aus der Literatur bekanntgeworden (siehe z. B. J. Org. Chem., 39, 245 [1974], J. Org. Chem. 41, 2855 [1976], J. Am. Chem. Soc., 86 5290 [1974], Ber., 97, 1298 [1964] sowie US-A-3 187 009 [Beispiel 2]). Die biologische Wirkung dieser Verbindungen ist noch nicht beschrieben worden. Die 2-Oxo-1,3-dithiol- und 2-Thioxo-1,3-dithiol-Verbindungen der Formel I sind neu und zeigen überraschenderweise eine den Pflanzenmetabolismus günstig beeinflussende Wirkung.

In den Verbindungen der Formel I sind unter Alkylresten verzweigte und geradkettige Alkylreste mit der angegebenen Zahl Kohlenstoffatome zu verstehen wie Methyl, Äthyl, n- und iso-Propyl, n-, sec-, iso- und tert.-Butylreste wie auch die geradkettigen und verzweigten Pentyl, Hexyl, Heptyl oder Octylreste. Dasselbe gilt für die Alkoxyreste. Der Alkylrest $R_1$ kann durch Halogen oder Phenyl substituiert oder durch Sauerstoffatome unterbrochen sein. Als Alkenyl- oder Alkinylreste $R_1$ kommen geradkettige oder verzweigte Reste mit 3—8 Kohlenstoffatomen in Frage. Bevorzugt sind der Allyl, Methallyl und der 2-Butenylrest sowie der Propinyl- und die Butinylreste. Die Phenylreste können unsubstituiert oder ein bis dreifach wie definiert substituiert sein. Als Cycloalkylreste kommen Cyclopropyl, Cyclobutyl, Cyclopentyl und Cyclohexyl in Frage.

Die 2-Oxo-1,3-dithiol- und 2-Thioxo-1,3-dithiol-Verbindungen der Formel I können auf verschiedene Arten hergestellt werden.

Diejenigen Verbindungen der Formel I, in denen $COOR_1$ und $R_2$ identische Carboxylester bedeutet, können gemäß L. R. Melby, H. D. Harzter und W. A. Sheppard, J. Org. Chem., 39, 245 (1974), durch Veresterung, in bekannter Weise, des bekannten 2-Thioxo-1,2-dithiol-3,4-dicarboxylates oder ihres 2-Oxohomoges (s. R. Mayer und B. Gebhardt in Ber., 97 [1964], 1298) mit einem Alkohol der Formel II hergestellt werden,

$$R_2—OH \qquad (II)$$

worin $R_2$ die oben angegebene Bedeutung hat, oder mit einem reaktionsfähigen Derivat (Mineralsäureester) eines solchen Alkohols.

Asymmetrische Verbindungen der Formel I, d. h. solche in denen $R_2$ nicht einen mit $COOR_1$ identischen Carboxylesterrest bedeutet, können hergestellt werden, indem man eine Verbindung der Formel III

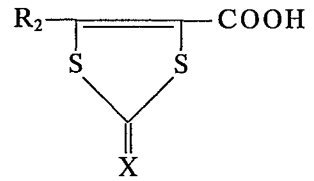

(III)

worin $R_2$ die in Formel I gegebene Bedeutung hat, in an sich bekannter Weise mit einem Alkohol der Formel II, oder einem reaktionsfähigen Derivat eines solchen Alkohols umsetzt, oder man überführt die Säure (III) in ein reaktionsfähiges Derivat (wie z. B. das Säurechlorid) und setzt dies in an sich bekannter Weise mit einem Alkohol der Formel II um.

Diese Veresterungen werden vorteilhafterweise bei Normaldruck und einer Temperatur, die zwischen Raumtemperatur und dem Siedepunkt des Reaktionsgemisches liegt, in einem inerten Lösungs- oder Verdünnungsmittel wie Benzol, Toluol, Äthylenchlorid, Chloroform oder einem Überschuß des Alkohols der Formel II vorgenommen. Es kann auch z. B. mittels Wasserabscheider gebildetes Kondenswasser aus der Reaktion destilliert werden, oder falls ein Mineralsäureester des Alkohols der Formel II eingesetzt wurde, in Gegenwart eines säurebindenden Mittels zum Ester umgesetzt werden. Die Verbindungen der Formel I können ferner hergestellt werden, indem man einen 2-Propynylcarbonsäureester der Formel IV mit einem Dithiocarbonat oder Trithiocarbonat der Formel V, gemäß untenstehendem Schema, bei mäßig erhöhter Temperatur umsetzt.

$R_2—C≡C—COOR_1$ (IV)

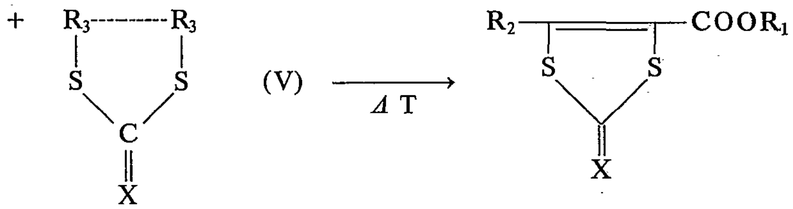


(V) (I)

In diesen Formeln haben $R_1$, $R_2$ und X die unter Formel I gegebene Bedeutung, $R_3$ ist $C_1-C_4$-Alkyl oder Benzyl oder die beiden $R_3$-Reste zusammen sind $C_1-C_4$-Alkylen.

Die Reaktion wird vorteilhafterweise in einem inerten Lösungsmittel, z. B. Toluol oder Xylol durchgeführt bei mäßig erhöhter Temperatur zwischen 50° und 150° (siehe dazu M. Narita und C. U. Pittmann, Synthesis, 1976, 496–514).

Schließlich können die asymmetrischen Verbindungen der Formel I, in denen $R_2$ keine Carboxylgruppe bedeutet, auch hergestellt werden, indem man eine Verbindung der Formel VI gemäß untenstehendem Schema in saurem Medium zu einer 2-Oxo-1,3-dithiol-Verbindung umsetzt oder mit Schwefelpentoxyd zu einer 2-Thioxo-1,3-dithiol-Verbindung umsetzt.

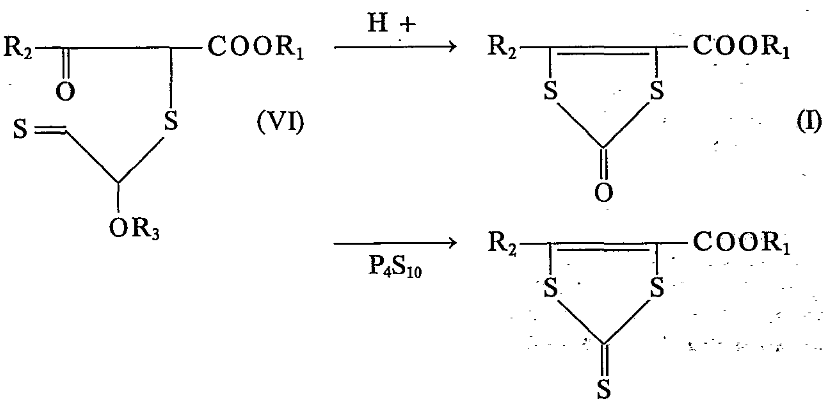


(VI) (I)

In diesen Formeln haben $R_1$, $R_2$ die unter Formel I gegebene Bedeutung, und $R_3$ ist ein $C_1-C_4$-Alkyl oder Benzylrest.

Diese Reaktionen werden entweder in starken wässerigen Säuren (z. B. 80% $H_2SO_4$), oder aber mit Phosphorpentasulfid in Dekalin vorgenommen, bei Temperaturen, die zwischen 30° C und 120° C liegen können.

2-Oxo-4,5-dicyano-1,3-thiol und das 2-Thioxo-4,5-dicyano-1,3-thiol der Formel VIII können gemäß einer Methode von E. Klingsberg, J. Am. Chem. Soc., 86, 5290 (1964), hergestellt werden, indem man das Natriumsalz von Dimercaptomaleinsäurenitril der Formel VII mit Phosgen oder Thiophosgen umsetzt.

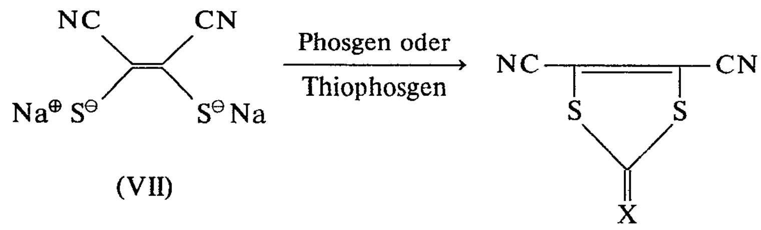


(VII) (VIII)

In Formel VIII bedeutet X Sauerstoff oder Schwefel. Diese Reaktion wird am besten in einem inerten Lösungsmittel wie Benzol oder Toluol bei einer Temperatur von 50°C bis 120°C durchgeführt. Diese Verbindungen können durch Verseifen der Nitrilgruppen und nachträgliche Veresterung zu Verbindungen der Formel I umgewandelt werden.

Die Verbindungen der Formel I sind unter normalen Umständen stabile, meist geruchlose, kristalline Verbindungen. Sie sind für Warmblüter wenig toxisch und können gefahrlos gehandhabt werden. Sie eignen sich vorzüglich zur Regulierung des Metabolismus und der Entwicklung von Pflanzen.

Darunter kommt vor allem die Förderung und Erleichterung der Fruchtabszission in Frage, vorzugsweise von Citrusfrüchten und Oliven, welche besonders schwierig und aufwendig zu pflücken sind, aber auch von anderen Früchten, die sich zum maschinellen Ernten eignen.

Der besondere Vorteil der Verbindungen vorliegender Erfindung besteht darin, daß bei deren Verwendung keinerlei phytotoxische Nebenerscheinungen an den Früchten, den Blättern oder anderen Pflanzenteilen auftreten.

Die Verbesserung der Fruchtabszission, d. h. die beträchtliche Reduzierung der beim manuellen und mechanischen Abernten aufzuwendenden Abreißkraft bringt große Vorteile und Erleichterungen bei der Ernte von Großkulturen mit sich, schont fruchttragende Bäume und Sträucher vor Beschädigung durch Mitabreißen von Zweigen und Blättern und spart Arbeitskräfte.

Es sind schon verschiedene Abszissionsmittel und Reifebeschleuniger in Vorschlag gebracht worden, die aber oft infolge unangenehmer Nebenwirkungen die Erfordernisse nicht befriedigen. So besitzt z. B. das »Cycloheximid«, β-[2-(3,5-Dimethyl-2-oxocyclohexyl)-2-hydroxyäthyl]-glutarimid, trotz hervorragender Abszissionswirkungen bei Citrus-Früchten den großen Nachteil, Blüten und noch unreife Früchte am Baum stark zu schädigen, auch stark entblätternd zu wirken und an reifen Früchten beträchtliche Narben zu erzeugen.

Als Reifebeschleuniger und Abszissionsmittel sind auch schon die β-Chloräthyl-phosphorsäure (»Ethrel®«, »Etephon«) oder andere Alkylen abgehende Mittel in Vorschlag gebracht worden, welche die in sie gesetzten Erwartungen wegen zu starker Defolierwirkung nicht immer erfüllen.

Die Wirkstoffe der Formel I sind in den üblichen Anwendungskonzentrationen nicht phytotoxisch und besitzen bloß eine geringe Warmblütertoxizität. Sie rufen keine morphologischen Veränderungen oder Schädigungen der Pflanzen hervor.

Zur Förderung der Fruchtabszission, insbesondere von Oliven und Citrusfrüchten, werden die fruchttragenden Bäume 3 Tage bis 4 Wochen vor der Ernte mit Mitteln behandelt, welche Wirkstoffe der obengenannten Formel enthalten.

Versuche ergaben weiter, daß mit den Verbindungen der Formel I bei Obstbäumen eine Blüten- und Fruchtausdünnung erfolgt. Bei vielen Zier- und Kulturpflanzen ist eine Steuerung von Blühtermin und Anzahl der Blüten möglich. Bei Ananas spielt dieser Effekt eine besonders große Rolle. Blühen alle Sträucher gleichzeitig, so kann das Abernten der Sträucher innerhalb einer vergleichsweise kurzen Zeit erfolgen. Bei Kürbisgewächsen erfolgt eine Verschiebung der Blütengeschlechtsdifferenzierung zugunsten der weiblichen Blüten.

Schließlich stimulieren die erfindungsgemäßen Verbindungen den Harzfluß der Pflanzen, was dort von Bedeutung ist, wo solche Harze wirtschaftlich genutzt werden wie bei der Latex-Gewinnung an Gummibäumen, oder der Harzgewinnung von Kiefern zur Terpentinölherstellung.

Die folgenden Beispiele beschreiben die Herstellung einiger neuer Verbindungen der Formel I. Weitere Verbindungen, die als Wirkstoffe verwendet werden können, finden sich in den nachfolgenden Tabellen. Die Temperaturen sind in Grad Celsius angegeben. Teile und Prozentangaben beziehen sich auf das Gewicht.

### Beispiel 1

#### Diäthyl-2-oxo-1,3-dithiol-4,5-dicarboxylat

Eine Lösung von 24 g (0,2 M) O,S-Äthylendithiocarbonat (s. F. N. Jones und S. Andreades, J. Org. Chem., 34, 3011, 1969) und 34 g (0,2 M) Acetylendicarbonsäurediäthylester in 100 ml Toluol wurden während 16 Stunden am Rückfluß gekocht. Nach dem Abkühlen wurde die Lösung bei vermindertem Druck eingeengt und das Rohprodukt auf Silicagel mit Äther zu Hexan = 1 : 3 chromatographiert. Man erhielt dabei die Verbindung der Struktur:

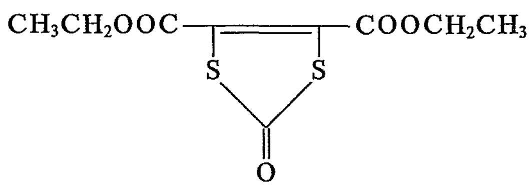

mit einem Brechungsindex $n_D^{20} = 1,5349$ als gelbliches Öl.

## Beispiel 2

### Diäthyl-2-thioxo-1,3-dithiol-4,5-dicarboxylat

Eine Lösung von 27,2 g (0,2 M) Äthylentrithiocarbonat und 34,0 g (0,2 M) Acetylendicarbonsäurediäthylester in 100 ml Toluol wurde während 6 Stunden am Rückfluß gekocht. Nach dem Abkühlen wurde die Lösung bei vermindertem Druck eingeengt und das Rohprodukt auf Silicagel mit Äther zu Hexan = 1 : 4 chromatographiert. Man erhielt dabei die Verbindung der Struktur:

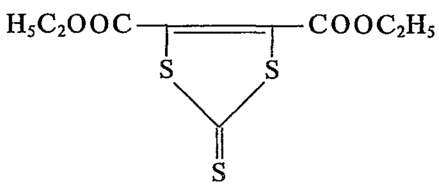

mit einem Brechungsindex $n_D^{20} = 1,6075$ als gelbliches Öl.

In analoger Weise wurde folgende Verbindungen hergestellt:

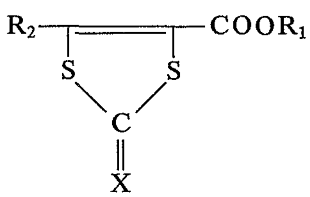

| $R_1$ | $R_2$ | X | Physikalische Konstante |
|---|---|---|---|
| $C_2H_5$ | $COOC_2H_5$ | O | $n_D^{20}$ 1,5349 Beispiel 1 |
| $C_2H_5$ | $COOC_2H_5$ | S | $n_D^{20}$ 1,6075 Beispiel 2 |
| $C_3H_7$-iso | $COOC_3H_7$-iso | S | $n_D^{20}$ 1,5799 |
| $C_5H_{11}$-n | $COOC_5H_{11}$-n | S | $n_D^{20}$ 1,5635 |
| $C_8H_{17}$-n | $COOC_8H_{17}$-n | S | $n_D^{20}$ 1,5404 |
| $C_3H_7$-iso | H | S | $n_D^{20}$ 1,6440 |
| $C_5H_{11}$-n | H | S | $n_D^{20}$ 1,6181 |
| $C_8H_{17}$-n | H | S | $n_D^{20}$ 1,5839 |
| $CH_2$— 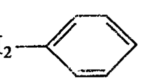 | H | S | Smp. 65–66° |
| 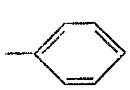 | H | S | Smp. 108–109° |
| $CH_2C{\equiv}CH$ | H | S | Smp. 65–66° |

Fortsetzung

| $R_1$ | $R_2$ | X | Physikalische Konstante |
|---|---|---|---|
| $CH_2CH{=}CH{-}CH_3$ | H | S | $n_D^{20}$ 1,6556 |
| $CH_2{-}C{\equiv}C{-}CH_3$ | H | S | Smp. 71–73° |
| $C_3H_6{-}CH{=}CH_2$ | H | S | $n_D^{20}$ 1,6343 |
| 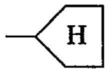 | H | S | Smp. 57–59° |
| 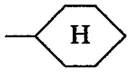 | H | S | $n_D^{20}$ 1,6432 |
| 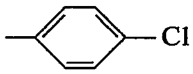 | H | S | Smp. 133–135° |
| 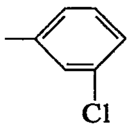 | H | S | Smp. 106–107° |
| 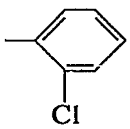 | H | S | Smp. 132–134° |
| 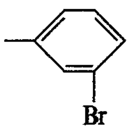 | H | S | Smp. 112–114° |
| 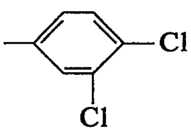 | H | S | Smp. 113–114° |
| 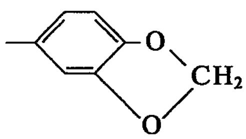 | H | S | Smp. 159–160° |
| 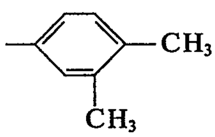 | H | S | Smp. 96–99° |
| 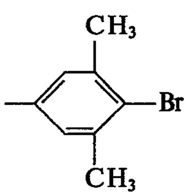 | H | S | Smp. 144–145° |
| 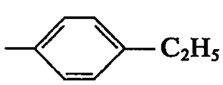 | H | S | Smp. 80–82° |
| 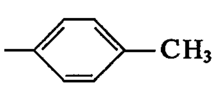 | H | S | Smp. 86–88° |

Fortsetzung

| $R_1$ | $R_2$ | X | Physikalische Konstante |
|---|---|---|---|
| 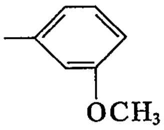 | H | S | Smp. 104–105° |
| 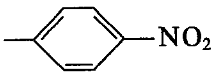 | H | S | Smp. 148–150° |
| 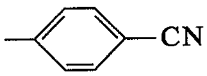 | H | S | Smp. 160–163° |
| 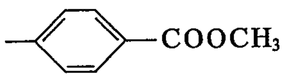 | H | S | Smp. 130–132° |
| 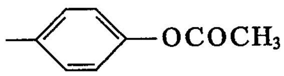 | H | S | Smp. 120–122° |
| 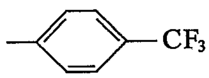 | H | S | Smp. 111–112° |
| 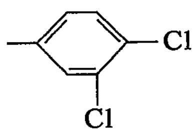 | $CH_3$ | O | Smp. 88–89° |
| $C_2H_5$ | $CH_3$ | S | $n_D^{20}$ 1,5556 |
| $CH_2—C\equiv CH$ | $CH_3$ | O | Smp. 73–75° |
| 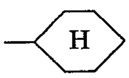 | $CH_3$ | O | $n_D^{20}$ 1,5561 |
| 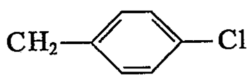 | $CH_3$ | O | Smp. 120–121° |
| 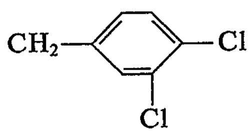 | $CH_3$ | O | Smp. 106–108° |
| 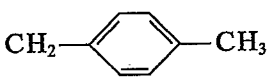 | $CH_3$ | O | Smp. 75–76° |
| 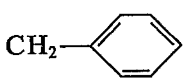 | H | S | Smp. 65–67° |
| 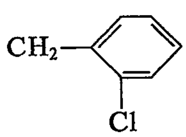 | H | S | Smp. 89–90° |
| 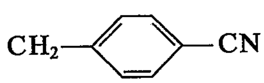 | H | S | Smp. 133–135° |
| 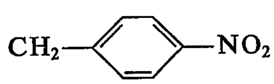 | H | S | Smp. 142–143° |

Fortsetzung

| $R_1$ | $R_2$ | X | Physikalische Konstante |
|---|---|---|---|
| 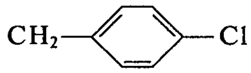 | H | S | Smp. 99–100° |
| 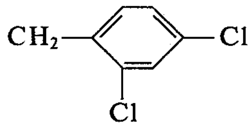 | H | S | Smp. 111–112° |
| 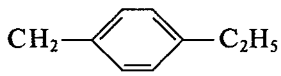 | H | S | $n_D^{20}$ 1,6529 |
| 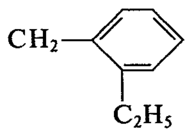 | H | S | $n_D^{20}$ 1,6529 |
| 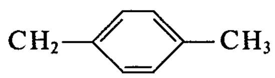 | H | S | Smp. 79–81° |
| 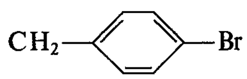 | H | S | Smp. 116–117° |
| 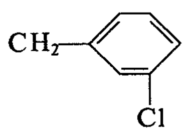 | H | S | Smp. 53–55° |
| 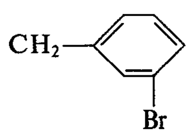 | H | S | Smp. 78–80° |
| 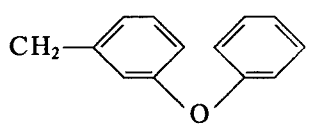 | H | S | Smp. 76–78° |
| $C_2H_5$ | 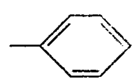 | O | Smp. 84–85° |
| $C_2H_4OC_3H_7$-iso | H | S | $n_D^{20}$ 1,6101 |
| $CH(CH_3)CH_2OCH_3$ | H | S | $n_D^{20}$ 1,6278 |
| $C_2H_5$ | 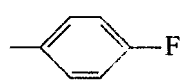 | O | Smp. 95–97° |
| 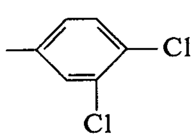 | 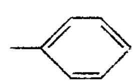 | O | Smp. 154–157° |
| $CH_3—C{\equiv}CH$ | $CH_3$ | O | Smp. 73–75 |
| 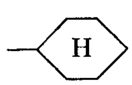 | $CH_3$ | O | $n_D^{20}$ 1,5561 |

Fortsetzung

| $R_1$ | $R_2$ | X | Physikalische Konstante |
|---|---|---|---|
| 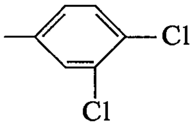 | CH | O | Smp. 98–99° |
| $C_4H_9$-n | $CH_3$ | O | $n_D^{20}$ 1,5366 |
| $C_3H_7$-iso | $CH_3$ | O | $n_D^{20}$ 1,5372 |
| 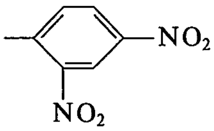 | $CH_3$ | O | Smp. 143–145° |
| $C_2H_4OCH_3$ | $CH_3$ | O | $n_D^{20}$ 1,5481 |
| $C_2H_4Cl$ | $CH_3$ | O | $n_D^{20}$ 1,5710 |
| $C_2H_4Br$ | $CH_3$ | O | $n_D^{20}$ 1,5919 |
| $C_3H_6Br$ | $CH_3$ | O | $n_D^{20}$ 1,5809 |
| 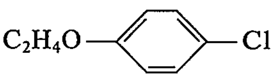 | H | S | Smp. 139–140° |
| 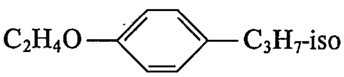 | H | S | $n_D^{20}$ 1,6108 |
| 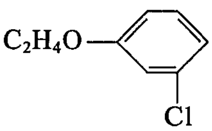 | H | S | $n_D^{20}$ 1,6676 |
| $(C_2H_4O)_2C_2H_5$ | H | S | $n_D^{20}$ 1,6088 |
| $(C_2H_4O)_2C_4H_9$-n | H | S | $n_D^{20}$ 1,5842 |
| $(C_2H_4O)_3CH_3$ | H | S | $n_D^{20}$ 1,5964 |
| 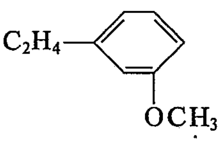 | H | S | Smp. 73–75° |
| 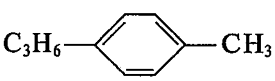 | H | S | Smp. 76–78° |
| 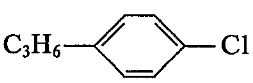 | H | S | Smp. 110–112° |
| 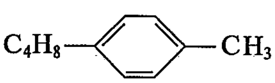 | H | S | Smp. 94–95° |
| 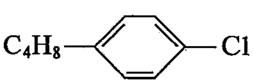 | H | S | Smp. 65–68° |
| 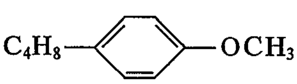 | H | S | Smp. 88–90° |
| $(C_2H_4O)_3C_2H_5$ | H | S | $n_D^{20}$ 1,5955 |

Fortsetzung

| $R_1$ | $R_2$ | X | Physikalische Konstante |
|---|---|---|---|
| $C_2H_5$ | 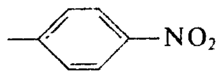 | O | Smp. 98–99° |
| 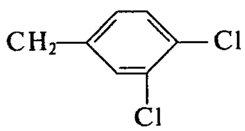 | 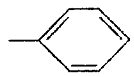 | O | Smp. 89–90° |

Die Verbindungen der Formel I vermögen den Pflanzenmetabolismus zu regulieren.

Sie fördern insbesondere die Fruchtausreifung und die Ausbildung von Trennungsgewebe, insbesondere zwischen Frucht und Fruchtstielen. Dadurch können Früchte aller Art, z. B. Steinobst (Oliven) und Citrusfrüchte, wie Orangen, Zitronen und Grapefruits ohne großen Kraftaufwand manuell oder maschinell von den Fruchtstielen abgelöst werden. Die üblicherweise beim Abernten durch Schütteln der Bäume und Sträucher sowie die beim Abreißen der Früchte auftretenden Verletzungen der Pflanzen am Blatt- und Astwerk werden dadurch weitgehend vermieden und so die Produktionskapazität der Bäume erhöht.

Das Ausmaß und die Art der Wirkung sind von verschiedensten je nach Pflanzenart variierenden Faktoren abhängig, insbesondere von der Anwendungskonzentration, dem Zeitpunkt der Anwendung in bezug auf das Entwicklungsstadium der Pflanze und den Früchten. So werden beispielsweise Pflanzen, deren Früchte verwendet resp. verwertet werden, unmittelbar nach der Blüte bzw. in entsprechendem Zeitabstand von der Ernte behandelt. Die Applikation erfolgt vorzugsweise in Form flüssiger Mittel sowohl auf oberirdische Pflanzenteile als auch in den Boden oder auf den Boden. Bevorzugt ist die Applikation auf die oberirdischen Pflanzenteile, für die sich Lösungen oder wäßrige Dispersionen am besten eignen.

Die Wirkstoffe der Formel I werden zusammen mit geeigneten Trägern, Lösungsmitteln und/oder anderen Zuschlagstoffen zur Anwendung gebracht. Geeignete Träger und Zuschlagstoffe können fest oder flüssig sein und entsprechen den in der Formulierungstechnik üblichen Stoffen, wie natürliche oder regenerierten mineralischen Stoffen, Lösungs-, Dispergier-, Netz-, Haft-, Verdickungs- oder Bindemitteln.

Die Aufwandmengen richten sich weitgehend nach dem Applikationszweck und der Art der Anwendung (Behandlung von Boden oder Pflanzenteilen). Die üblichen Aufwandmengen bei Bodenbehandlung und Flächenkulturen liegen zwischen 0,1 und 10 kg Wirkstoff pro Hektar Pflanzenbestand, vorzugsweise 0,4 bis 4 kg Wirkstoff pro Hektar.

Die Mittel zur Abszission und Reifebeschleunigung, welche Wirkstoffe der Formel I enthalten, können als wässerige oder nichtwässerige Lösungen und Dispersionen, emulsitierbare Konzentrate, Spritzpulver oder als Stäubemittel, gegebenenfalls unter Zusatz von Antioxydantien wie z. B. Hydrochinon, formuliert werden. Solche Formulierungen können 2 bis 95 Gew.-%, vorzugsweise 80–90 Gew.-% Wirkstoff enthalten und nach den in der Agrikulturchemie üblichen Techniken hergestellt werden.

Bevorzugt sind wässerige Aufbereitungen mit einem Zusatz von 0,01 bis 1% an einem nichtionischen Netzmittel.

Der Zeitpunkt der Anwendung liegt für Fruchtabszission kurz vor dem Abernten, d. h. 3 Tage bis zu 4 Wochen vor der Ernte, für Reifebeschleunigung kurz vor oder nach dem Abernten der Früchte.

Die Bestimmung der Abszissionswirkung bei Citrus-Pflanzen erfolgte nach folgenden Methoden:

Astpartien von Orangen-Bäumen (Sorte Navel) mit mindestens 20 Früchten wurden kurz vor der Ernte mit Wirkstofflösungen bespritzt. Nach 7 Tagen erfolgte die Auswertung, wobei zwei verschiedene Systeme verwendet wurden:

a) Messung der Abreißkraft und ihre prozentuale Minderung in Relation zur unbehandelten Kontrolle.
b) Anzahl abgefallener Früchte ohne Schütteln in Prozent, verglichen mit unbehandelter Kontrolle.

Alle untersuchten Wirkstoffe zeigten bei keinem oder nur geringem Blattfall starke Ausbildung von Trennungsgeweben an den Fruchtstielen, merkliche Reduktion der Abreißkraft und manche sogar gute Werte im Fruchtfall.

| Wirkstoff | Anwendungs-konzentration | Reduktion der Abreißkraft | Gefallene Orangen |
|---|---|---|---|
| Beisp. 1 | 4000 ppm | 60% | 40% |
| Beisp. 1 | 2000 ppm | 44% | 33% |
| Beisp. 1 | 1000 ppm | 19% | 22% |
| — | Kontrolle | 0% (8,9 kg) | 0% |

Bei Olivenbäumen wurden ähnliche Versuche angestellt. Astpartien wurden 8 Tage vor dem erwarteten Erntezeitpunkt mit einer stark verdünnten Wirkstofflösung besprüht, wobei man gleichzeitig die Anzahl Oliven in der Astpartie zählte. Ähnlich große Astpartien am selben Baum ließ man unbehandelt zwecks Kontrolle der Wirkung. Nach 8 Tagen wurden die Astpartien gleichmäßig von Hand geschüttelt. Während bei den behandelten Astpartien 90—100% der Oliven heruntergeschüttelt werden konnten, blieben bei den unbehandelten Kontrollen 30—60% der Früchte am Ast hängen.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| in Wasser dispergierbare Wirkstoffkonzentrate: | Spritzpulver (wettable powder), emulgierbare Konzentrate; |
| flüssige Aufarbeitungsformen: | Lösungen. |

Zur Herstellung fester Aufbereitungsformen (Pasten, Stäubemittel, Streumittel, Granulate) werden die Wirkstoffe mit festen Trägerstoffen vermischt. Als Trägerstoffe kommen zum Beispiel Kaolin, Talkum, Bolus, Löß, Kreide, Kalkstein, Kalkgrits, Taclay, Dolomit, Diatomeenerde, gefällte Kieselsäure, Erdalkalisilikate, Natrium- und Kaliumaluminiumsilikate (Feldspate und Glimmer), Calcium- und Magnesiumsulfate, Magnesiumoxid, gemahlene Kunststoffe, Düngemittel, wie Ammoniumsulfat, Ammoniumphosphat, Ammoniumnitrat, Harnstoff, gemahlene pflanzliche Produkte, wie Getreidemehl, Baumrindenmehl, Holzmehl, Nußschalenmehl, Cellulosepulver, Rückstände von Pflanzenextraktionen und Aktivkohle, je für sich oder als Mischungen untereinander in Frage.

Die Korngröße der Trägerstoffe beträgt für Stäubemittel zweckmäßig bis 0,1 mm, für Streumittel 0,075 bis 0,2 mm und für Granulate 0,2 mm oder mehr.

Die Wirkstoffkonzentrationen in den festen Aufarbeitungsformen betragen 0,5 bis 80%.

Diesen Gemischen können ferner den Wirkstoff stabilisierende Zusätze und/oder nichtionische, anionenaktive und kationenaktive Stoffe zugegeben werden, die beispielsweise die Haftfestigkeit der Wirkstoffe auf Pflanzen und Pflanzenteilen verbessern (Haft- und Klebemittel) und/oder eine bessere Benetzbarkeit (Netzmittel) sowie Dispergierbarkeit (Dispergatoren) gewährleisten. Als Klebemittel kommen beispielsweise die folgenden in Frage: Olein-Kalk-Mischung, Cellulosederivate (Methylcellulose, Carboxymethylcellulose), Hydroxyäthylenglykoläther von Mono- und Dialkylphenolen mit 5—15 Äthylenoxidresten pro Molekül und 8—9 Kohlenstoffatomen im Alkylrest, Ligninsulfonsäure, deren Alkali- und Erdalkalisalze, Polyäthylenglykoläther (Carbowaxe), Fettalkoholpolyglykoläther mit 5—20 Äthylenoxidresten pro Molekül und 8—18 Kohlenstoffatomen im Fettalkoholteil, Kondensationsprodukte von Äthylenoxid, Propylenoxid, Polyvinylpyrrolidine, Polyvinylalkohole, Kondensationsprodukte von Harnstoff-Formaldehyd sowie Latex-Produkte.

In Wasser dispergierbare Wirkstoffkonzentrate, d. h. Spritzpulver (wettable powder), Pasten und Emulsionskonzentrate stellen Mittel dar, die mit Wasser auf jede gewünschte Konzentration verdünnt werden können. Sie bestehen aus Wirkstoff, Trägerstoff, gegebenenfalls den Wirkstoff stabilisierenden Zusätzen, oberflächenaktiven Substanzen und Antischaummitteln und gegebenenfalls Lösungsmitteln. Die Wirkstoffkonzentration in diesen Mitteln beträgt 5—80%.

Die Spritzpulver (wettable powder) und Pasten werden erhalten, indem man die Wirkstoffe mit Dispergiermitteln und pulverförmigen Trägerstoffen in geeigneten Vorrichtungen bis zur Homogenität vermischt und vermahlt. Als Trägerstoffe kommen beispielsweise die vorstehend für die festen Aufarbeitungsformen erwähnten in Frage. In manchen Fällen ist es vorteilhaft, Mischungen verschiedener Trägerstoffe zu verwenden. Als Dispergatoren können beispielsweise verwendet werden: Kondensationsprodukte von sulfoniertem Naphthalin und sulfonierten Naphthalinderivaten mit Formaldehyd, Kondensationsprodukte des Naphthalins bzw. der Naphthalinsulfonsäuren mit

Phenol und Formaldehyd sowie Alkali-, Ammonium- und Erdalkalisalze von Ligninsulfonsäure, weiter Alkylarylsulfonate, Alkali- und Erdalkalimetallsalze der Dibutylnaphthalinsulfonsäure, Fettalkoholsulfate, wie Salze sulfatierter Hexadecanole, Heptadecanole, Octadecanole und Salze von sulfatiertem Fettalkoholglykoläther, das Natriumsalz von Oleylmethyltaurid, ditertiäre Acetylenglykole, Dialkyldilaurylammoniumchlorid und fettsaure Alkali- und Erdalkalisalze.

Als Antischaummittel kommen zum Beispiel Silicone in Frage.

Die Wirkstoffe werden mit den oben aufgeführten Zusätzen so vermischt, vermahlen, gesiebt und passiert, daß bei den Spritzpulvern der feste Anteil eine Korngröße von 0,02 bis 0,04 und bei den Pasten von 0,03 mm nicht überschreitet. Zur Herstellung von Emulsionskonzentraten und Pasten werden Dispergiermittel, wie sie in den vorangehenden Abschnitten aufgeführt wurden, organische Lösungsmittel und Wasser verwendet. Als Lösungsmittel kommen beispielsweise die folgenden in Frage: Alkohole, Benzole, Xylole, Toluol, Dimethylsulfoxid und im Bereich von 120° bis 350° siedende Mineralölfraktionen. Die Lösungsmittel müssen praktisch geruchlos, nicht phytotoxisch, den Wirkstoffen gegenüber inert und dürfen nicht leicht brennbar sein.

Ferner können die erfindungsgemäßen Mittel in Form von Lösungen angewendet werden. Hierzu wird der Wirkstoff bzw. werden mehrere Wirkstoffe der allgemeinen Formel I in geeigneten organischen Lösungsmitteln, Lösungsmittelgemischen oder Wasser gelöst. Als organische Lösungsmittel können aliphatische und aromatische Kohlenwasserstoffe, deren chlorierte Derivate, Alkylnaphthaline, Mineralöle allein oder als Mischung untereinander verwendet werden. Die Lösungen sollen die Wirkstoffe in einem Konzentrationsbereich von 1 bis 20% enthalten.

Den beschriebenen erfindungsgemäßen Mitteln lassen sich andere biozide Wirkstoffe oder Mittel beimischen. So können die neuen Mittel außer den genannten Verbindungen der allgemeinen Formel I zum Beispiel Insektizide, Fungizide, Bakterizide, Fungistatika, Bakteriostatika oder Nematozide zur Verbreiterung des Wirkungsspektrums enthalten. Die erfindungsgemäßen Mittel können ferner noch Pflanzendünger und Spurenelemente enthalten.

Im folgenden werden Aufarbeitungsformen der neuen Wirkstoffe der allgemeinen Formel I beschrieben. Teile bedeuten Gewichtsteile.

## Spritzpulver

Zur Herstellung eines a) 50%igen, b) 25%igen und c) 10%igen Spritzpulvers werden folgende Bestandteile verwendet:

a) 50 Teile Diäthyl-2-thioxo-1,3-dithiol-4,5-dicarboxylat,
5 Teile Natriumdibutylnaphthylsulfonat,
3 Teile Naphthalinsulfonsäuren-Phenolsulfonsäuren-Formaldehyd-Kondensat 3 : 2 : 1,
20 Teile Kaolin,
22 Teile Champagne-Kreide;

b) 25 Teile des obigen Wirkstoffes,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin;

c) 10 Teile des obigen Wirkstoffes,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschließend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden.

## Paste

Zur Herstellung einer 45%igen Paste werden folgende Stoffe verwendet:

45 Teile Di-isopropyl-2-oxo-1,3-dithiol-4,5-dicarboxylat,
5 Teile Natriumaluminiumsilikat,
14 Teile Cetylpolyglykoläther mit 8 Mol Äthylenoxid,

1 Teil Oleylpolyglykoläther mit 5 Mol Äthylenoxid,
2 Teile Spindelöl,
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile Dibenzyl-2-thioxo-1,3-dithiol-4,5-dicarboxylat,
5 Teile Mischung von Nonylphenolpolyoxyläthylen und Calcium-dodecylbenzol-sulfonat,
35 Teile 3,5,5-Trimethyl-2-cyclohexen-1-on,
35 Teile Dimethylformamid

miteinander vermischt. Dieses Konzentrat kann mit Wasser zu Emulsionen auf geeignete Konzentrationen verdünnt werden.

**Patentansprüche**

1. 2-Oxo- und 2-Thioxo-1,3-dithiol-Verbindungen der Formel I

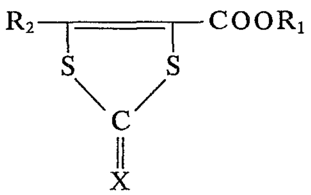

(I)

worin
$R_1$ $C_1-C_8$-Alkyl, gegebenenfalls ein- oder mehrfach durch Sauerstoff unterbrochen oder substituiert durch Halogen, Phenyl oder Phenoxy, wobei der Phenylkern durch Halogen, Cyano, Nitro, Trifluormethyl, $C_1-C_4$-Alkyl, $C_1-C_4$-Alkoxy oder Phenoxy substituiert sein kann;
$C_3-C_8$ Alkenyl, gegebenenfalls substituiert durch Halogen;
$C_3-C_8$ Alkinyl;
$C_3-C_6$ Cycloalkyl;
Phenyl, gegebenenfalls substituiert durch Halogen, Cyano, Nitro, Trifluormethyl, $C_1-C_4$-Alkyl, $C_1-C_4$-Alkoxy oder Phenoxy, $C_1-C_4$-Alkoxycarbonyl, Acetyloxy- oder Dioxy-$C_1-C_2$-alkylen;
$R_2$ Wasserstoff, Methyl; $C_1-C_8$-Alkoxycarbonyl oder Phenyl, gegebenenfalls substituiert durch Halogen, Cyano, Nitro, Trifluormethyl, $C_1-C_4$-Alkyl, $C_1-C_4$-Alkoxy oder Phenoxy;
X Sauerstoff oder Schwefel, bedeuten, mit der Maßgabe, daß wenn $R_1$ Methyl ist, $R_2$ nicht Wasserstoff oder Methoxycarbonyl sein kann.

2. Den Pflanzenmetabolismus regulierendes Mittel, welches als Wirkstoff mindestens eine 2-Oxo- oder 2-Thioxo-1,3-dithiol-Verbindung der Formel I, Anspruch 1, enthält.
3. Den Pflanzenmetabolismus regulierendes Mittel, welches als Wirkstoff Diäthyl-2-oxo-1,3-dithiol-4,5-dicarboxylat enthält.
4. Die Verwendung der Verbindungen der Formel I, Anspruch 1, oder sie enthaltender Mittel zur Regulierung des Pflanzenmetabolismus.
5. Die Verwendung der Verbindungen der Formel I, Anspruch 1, oder sie enthaltender Mittel zur Förderung der Abszission von erntereifen Früchten, vor allem von Citrusfrüchten und Oliven.
6. Verfahren zur Herstellung der Verbindungen der Formel I, Anspruch 1, dadurch gekennzeichnet, daß man bei mäßig erhöhter Temperatur zwischen 50°C und 150°C in einem inerten Lösungsmittel einen 2-Propinyl-carbonsäureester der Formel IV

$$R_2-C\equiv C-COOR_1 \qquad (IV)$$

worin $R_1$ und $R_2$ die unter Formel I gegebene Bedeutung haben, mit einem Dithiocarbonat oder Trithiocarbonat der Formel V umsetzt,

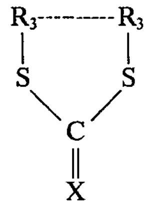

(V)

worin X Sauerstoff oder Schwefel und $R_3$ $C_1-C_4$-Alkyl oder Benzyl oder die beiden $R_3$-Reste zusammen $C_1-C_4$-Alkylen bedeuten.

**Claims**

1. A 2-oxo- or 2-thioxo-1,3-dithiol compound of the formula I

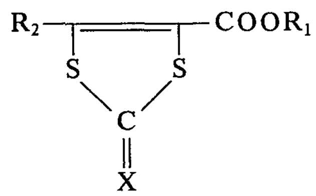

(I)

wherein

$R_1$ is $C_1-C_8$ alkyl which can be interrupted by one or more oxygen atoms or substituted by halogen, phenyl or phenoxy, whilst the phenyl nucleus can be substituted by halogen, cyano, nitro, trifluoromethyl, $C_1-C_4$ alkyl, $C_1-C_4$ alkoxy or phenoxy;
$C_3-C_8$ alkenyl which is unsubstituted or substituted by halogen;
$C_3-C_8$ alkynyl;
$C_3-C_6$ cycloalkyl;
phenyl which is unsubstituted or substituted by halogen, cyano, nitro, trifluoromethyl, acetyloxy or dioxy-$C_1-C_2$-alkylene;

$R_2$ is hydrogen, methyl, $C_1-C_8$ alkoxycarbonyl, or phenyl which is unsubstituted or substituted by halogen, cyano, nitro, trifluoromethyl, $C_1-C_4$ alkyl, $C_1-C_4$ alkoxy or phenoxy;

X is oxygen or sulfur; with the proviso that, if $R_1$ is methyl, $R_2$ may not be hydrogen or methoxycarbonyl.

2. A composition for regulating plant metabolism which contains, as active ingredient, at least one 2-oxo- or 2-thioxo-1,3-dithiol compound of the formula I according to claim 1.

3. A composition for regularing plant metabolism, which contains, as active ingredient, diethyl-2-oxo-1,3-dithiol-4,5-dicarboxylate.

4. A method of regulating plant metabolism which comprises the use of a compound of the formula I according to claim 1, or of a composition containing such a compound.

5. A method of promoting the abscission of fruit which is ripe for harvesting, in particular citrus fruit and olives, which comprises the use of a compound of the formula I according to claim 1 or of a composition containing such a compound.

6. A process for the manufacture of a compound of the formula I according to claim 1, which process comprises reacting a 2-propynylcarboxylate of the formula IV

$$R_2—C\equiv C—COOR_1 \qquad (IV)$$

wherein $R_1$ and $R_2$ are as defined for formula I, at moderately elevated temperature in the range from 50° to 150° C and in an inert solvent, with a dithiocarbonate or trithiocarbonate of the formula V

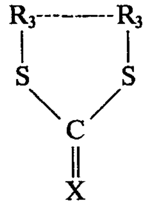

(V)

wherein X is oxygen or sulfur and $R_3$ represents $C_1-C_4$ alkyl, or benzyl, or both radicals $R_3$ together are $C_1-C_4$ alkylene.

**Revendications**

1. Composés de 2-oxo- et de 2-thioxo-1,3-dithiol de formule I

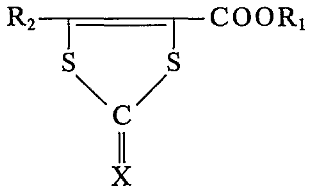

(I)

où

$R_1$ représente un alcoyle en $C_1$ à $C_8$ éventuellement mono- ou poly-interrompu par un oxygène ou substitué par un halogène, un phényle ou un phénoxy, où le noyau phényle peut être substitué par un halogène, un cyano, un nitro, un trifluorométhyle, un alcoyle en $C_1$ à $C_4$, un alcoxy en $C_1$ à $C_4$ ou un phénoxy;
un alcényle en $C_3$ à $C_8$ éventuellement substitué par un halogène;
un alcynyle en $C_3$ à $C_8$;
un cycloalcoyle en $C_3$ à $C_6$;
un phényle, eventuellement substitué par un halogène, un cyano, un nitro, un trifluorométhyle, un alcoyle en $C_1$ à $C_4$, un alcoxy en $C_1$ à $C_4$ ou un phénoxy, un alcoxycarbonyle en $C_1$ à $C_4$, un acétyloxy ou un dioxy-alcoylène en $C_1$ à $C_2$;

$R_2$ représente un hydrogène, un méthyle, un alcoxycarbonyle en $C_1$ à $C_8$ ou un phényle, éventuellement substitué par un halogène, un cyano, un nitro, un trifluorométhyle, un alcoyle en $C_1$ à $C_4$, un alcoxy en $C_1$ à $C_4$ ou un phénoxy;

X représente un oxygène ou un soufre, avec la précision que lorsque $R_1$ est un méthyle, $R_2$ ne peut pas être un hydrogène ou un méthoxycarbonyle.

2. Agent régulateur du métabolisme des plantes contenant comme matière active au moins un composé de 2-oxo- ou de 2-thioxo-1,3-dithiol de formule I selon la revendication 1.

3. Agent régulateur du métabolisme des plantes contenant comme matière active le diéthyl-2-oxo-1,3-dithiol-4,5-dicarboxylate.

4. Application des composés de formule I de la revendication 1 ou des agents qui les contiennent à la régulation du métabolisme des plantes.

5. Application des composés de formule I de la revendication 1 ou des agents qui les contiennent à la facilitation de la séparation des fruits mûrs pour la cueillette, surtout les agrumes et les olives.

6. Procédé de préparation des composés de formule I de la revendication 1, caractérisé en ce qu'on fait réagir à une température modérément augmentée entre 50° C et 150° C dans un solvant inerte un ester d'acide 2-propinylcarboxylique de formule IV

$$R_2—C\equiv C—COOR_1 \qquad (IV)$$

où $R_1$ et $R_2$ ont la signification donnée pour la formule I, avec un dithiocarbonate ou trithiocarbonate de formule V

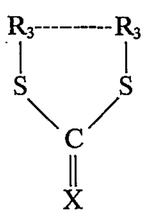

(V)

où X représente un oxygène ou un soufre et $R_3$ un alcoyle en $C_1$ à $C_4$ ou un benzyle, ou bien où les deux radicaux $R_3$ forment ensemble un alcoylène en $C_1$ à $C_4$.